# EUROPEAN PATENT APPLICATION

(11) **EP 4 008 249 A1**
(43) Date of publication of application: **08.06.2022**
(21) Application number: 20211521.8
(22) Date of filing: 03.12.2020
(51) Int. Cl.: A61B 5/06, A61B 90/00, A61B 34/20, A61M 25/01, A61B 17/00

(54) **ULTRASOUND-ACTIVATED WIRELESS MICRO-MAGNETIC MARKER FOAM PATCH**

(71) Applicant: Koninklijke Philips N.V., 5656 AG Eindhoven (NL)
(72) Inventor: GLEICH, Bernhard, 5656 AE Eindhoven (NL); RAHMER, Jürgen Erwin, 5656 AE Eindhoven (NL)
(74) Representative: Philips Intellectual Property & Standards

(57) **Abstract**

A marker device to be tracked by a respective reading system is provided, which allows to track a position of a medical device with high accuracy using a magnetic field that is generated through movement of a plurality of magnetic objects in a foam-like medium. For this purpose, the foam-like medium is caused to oscillate using an externally applied excitation field, in particular an ultrasound excitation field. The movement of the plurality of magnetic objects causes a change in the magnetic field caused by the magnetic objects. This change may be detected by a respective magnetic response detector and used to determine the position of the marker device and, hence, the medical device.

## Description

### FIELD OF THE INVENTION

The invention relates to a marker device, a reading system for tracking a marker device, a method for manufacturing a marker device and a method for tracking a marker device.

### BACKGROUND OF THE INVENTION

For certain invasive, and in particular minimally invasive, medical procedures, such as local anesthesia, it may be beneficial to track a medical device inserted into the human body, such as a needle used for applying the local anesthesia. This tracking should hereby be as accurate as possible.

One approach to track a medical device used for such procedures using ultrasound imaging. Hereby, the medical device is equipped with an ultrasound transducer and tracked by a respective ultrasound detector. The ultrasound detector may be used to generate an ultrasound image, indicating the position of the medical device. In some approaches, the ultrasound transducer may particularly be a transducer implementing piezoelectric polyvinylidene fluoride (PVDF) films as transducer material. Employing PVDF films as transducer material this allows for broad bandwidth and focusing action. Further kinds of ultrasound transducers may also be used.

For detection of the ultrasound waves emitted by the transducer, a respective ultrasound detector has to be used. Such ultrasound detectors are typically provided with a cable connection in order to read out the detector. For procedures such as local anesthesia, the handling of ultrasound detectors which a cable connection is both, cumbersome and relatively expensive.

On the other hand, more simple and less expensive methods of tracking lack the accuracy that is needed in certain procedures, such as local anesthesia.

### SUMMARY OF THE INVENTION

It is therefore an object of the present invention to overcome the above indicated short-comings. In particular, it is an object of the invention to provide an approach which allows to perform tracking in a very accurate manner while at the same time keeping the costs incurred by such tracking relatively low. Even more specifically, it is an object of the invention to provide an approach that employs components that may be manufactured at low costs, may provide for high tracking efficiency and which use is simple and easy to perform.

According to a first aspect of the invention, this object is solved by a marker device to be tracked, wherein the marker device comprises a body comprising a foam-like medium and at least one magnetic object arranged in contact with the foam-like medium, wherein the foam-like medium is adapted to oscillate in response to an externally applied excitation field, such as to initiate a motion of the at least one magnetic object.

In this context, the marker device may particularly be provided in the format of a miniature patch that may be attached to the medical device. The miniature patch may hereby particularly have a body. The term body may, in this context, be understood as the main part of the marker device that forms the miniature patch.

The body may comprise and/or consist of a foam-like medium. That is, is some embodiments, the foam-like medium may be provided in the entire body of the marker device. In some embodiments, the body of the marker device may be larger than the part of the body comprising the foam-like medium.

The term foam-like medium hereby has to be understood broadly as any kind of material having a flexible and/or viscous structure and one or more holes or the like therein. In particular, the term foam-like medium may refer to a flexible solid material, such as a rubber, having respective holes or bubbles formed therein and/or a liquid material capable of forming foam which allows to form holes or bubbles of a given size. The holes or bubbles shall be capable of oscillating in response to an externally applied excitation field, thereby acting as oscillation enhancers in the foam-like medium. In some embodiments, the foam-like medium may particularly be made of a material in which the holes or bubbles formed have a predetermined size, i.e. a size within a particular range.

The term magnetic object may particularly refer to a magnetic object having a size such a plurality of magnetic objects may attach onto the holes or bubbles formed by the foam. In some embodiments, the term magnetic object may particularly refer to magnetic beads. In some embodiments, the magnetic object may be derived from a magnetic powder. The magnetic object may be arranged in contact with the foam-like medium. In some embodiments, a plurality of magnetic objects may be used. This plurality of magnetic objects may be dispersed through the foam-like medium. In some embodiments, this dispersion causes the plurality of magnetic objects to attach on close to the holes or bubbles provided therein. As an example, the magnetic objects may attach and/or be provided in the free space of the holes in the foam-like medium. As another example, the magnetic objects may attach and/or be provided on the surfaces of the bubbles in the foam-like medium.

The term externally applied excitation field may particularly refer to an ultrasound excitation field that is applied on the marker device by a respective ultrasound generator. The externally applied excitation field causes the foam-like medium to respond to the excitation field by a respective oscillation.

As such, the present approach shows some similarities to known approaches in which PVDF is used as an ultrasound transducer. Contrary to the approach using PVDF, there is no cable connection needed for the present approach, making it much more flexible and less costly. Hereby, the present approach is based on the understanding that the tracking may be performed by providing a marker device comprising a foam-like medium in which at least one magnetic object, particularly a plurality of magnetic objects, such as magnetic beads, is distributed.

In essence, the marker device may then be tracked by using the external ultrasound excitation field to initiate motion, in particular tilting, of the at least one magnetic obj ect.

Hereby, in order to have a sufficiently good response to the externally applied excitation field, holes, bubbles, such as gas bubbles, or the like as a form of oscillation enhancers may be provided in the foam-like medium. This allows to enhance the effect on the magnetic object even for relatively small excitation fields as the oscillation enhancers are caused to oscillate in response to the excitation field, thereby enhancing motion of the magnetic objects. For this purpose, the foam-like medium may particularly be provided such as to contain a plurality of oscillation enhancers of a particular size, whereby this size is such that the oscillation frequency of the oscillation enhancers corresponds to the resonance frequency of the excitation field. This further enhances the response to the excitation field generated by the ultrasound wave, thereby increasing the motion of the at least one magnetic obj ect.

That is, when an external excitation field (e.g. by an ultrasound frequency) is applied, the foam-like medium responds to this by having the oscillation enhancers therein oscillate according to the frequency of the excitation field. This causes the magnetic object or magnetic objects to be moved, particularly to be tilted.

The motion, specifically tilting motion, causes a change in the magnetic field caused by the at least one magnetic object in the marker device. This change in magnetic field may then be read out by a reading system. The reading system may thus detect the marker device on the basis of the magnetic field. This allows tracking the marker device with relatively high accuracy.

For this purpose, the magnetic object or magnetic objects may particularly correspond to a hard magnetic object, i.e. a magnetic object having a fixed dipole moment. Using magnetic objects with a fixed dipole moment results in the change in magnetic field being caused solely by the motion of the magnetic object, whereas no change in magnetic properties may influence the accuracy.

In some embodiments, however, it may also be possible to use a combination of a hard magnetic material and a soft magnetic material in the foam-like medium, i.e. to provide a first plurality of magnetic objects comprising or being made of a hard magnetic material and a second plurality of magnetic objects comprising or being made of a soft magnetic material. The use of such a first plurality of magnetic objects comprising or being made of a hard magnetic material and a second plurality of magnetic objects comprising or being made of a soft magnetic material may allow to employ the effect of correlated magnetization change, thereby allowing to derive further information on the marker device. In this context, correlated magnetization change means that if the marker device, being made of a foam-like, and, thus, flexible, medium is, for example, squeezed together, the magnetization is increases as the flux lines are better concentrated in the more dense material.

However, in such cases, it would, be necessary to also provide, in addition to the external excitation field caused by the ultrasound generator, a magnetic reference field. This magnetic reference field may act as a reference, in order to distinguish the components of the magnetic field detected, such as to determine which change is caused by motion of the magnetic objects and which change in magnetic field is caused by the change in magnetic properties.

This magnetic reference field may hereby be generated in a plurality of manners. In some embodiments, the magnetic reference field may be an externally generated field, generated by an external magnetic field generator. However, using such an external magnetic field generator may be complicated. Thus, in some embodiments, a permanent magnet may be used to generate the magnetic reference field. This permanent magnet may be very small and may be provided at or near the marker device itself. Alternative or additionally, the magnetic reference field may be generated by the first plurality of magnetic objects comprising or being made of the hard magnetic material. As indicated herein above, these objects are caused to move, in particular tilt, thereby generating and changing a magnetic field. This may be sufficient in itself to provide the necessary reference.

In accordance with the current approach, a marker device is provided which allows to track the position of a medical device with high accuracy. This tracking is hereby performed by measuring a change in magnetic field that is caused by the motion of magnetic objects, such as magnetic beads and/or magnetic particles in a foam-like medium that is caused to oscillate, in particular at the resonance frequency, in response to an external excitation field caused by the ultrasound waves generated by an ultrasound generator.

In some embodiments, the foam-like medium may comprise one or more oscillation enhancers having a predetermined size.

As discussed herein above, the foam-like medium may particularly comprise oscillation enhancers. The term oscillation enhancer may hereby refer to any kind of measure in the foam-like medium that enhances the oscillation in response to the external excitation field. In some embodiments, the oscillation enhancers may particularly correspond to holes and/or bubbles, in particular gas bubbles, that are provided in a foam-like medium.

In some embodiments, the oscillation enhancers may particularly have a predetermined size. This size is predetermined in that it is determined to correspond to a size such that the oscillation frequency of the oscillation objects is close to the applied ultrasound frequency.

Hereby, it shall be understood that the term predetermined size does not refer to one single particular size, but that the size may be variable based on the material used for the foam-like medium. Further, in one marker device, the oscillation enhancers may have different sizes within a particular size range, thereby also having said predetermined size. That is, it shall be understood that the predetermined size may particularly comprise multiple size values in a particular range.

The predetermined size may be determined by means of computation. As an example, it may be assumed that, there would be only a single oscillation enhancer in the in the foam-like medium. The material of the foam-like medium and the oscillation enhancer therein is known. Further, the frequency of the excitation field caused by the ultrasound wave is known. Then, it may be determined which size the oscillation enhancer should have in order to oscillate with the resonance frequency of the external excitation field.

Typically, however, the foam-like medium may comprise a plurality of oscillation enhancers. These oscillation enhancers may be of a particular predetermined size, i.e. may have size values within the predetermined range. It is noted that in this case, a highly coupled system is provided in which the response to the external excitation field of one oscillation enhancer is coupled to the response of one or more other oscillation enhancers. Hereby, as indicated above, the size values of the multiple oscillation enhancers need not be exactly the same. However, the distribution of size values of the individual oscillation enhancers alone with their respective proximity, surface tension, the density of the foam-like medium and the like need to be in the required range.

In some embodiments, the particular combination of values for these features may particularly be determined using experimental measurements. For this purpose, different materials forming the basis of the foam-like medium and different materials for the at least one magnetic object may be tested.

In some embodiments, the foam-like medium may be provided by mixing a liquid with a foaming agent. Further, the magnetic objects may be added to the mixture. The resulting composition may then be stirred, in particular whipped, to generate the foam-like medium comprising the at least one magnetic object. During the whipping, the magnetic objects are dispersed through the liquid-foaming agent mixture. Further, the liquid foams such as to form the foam-like medium. That is, the oscillation enhancers, such as respective bubbles in the foam, may be formed. The magnetic objects may hereby preferably be provided such as to concentrate on the surfaces of the oscillation enhancers.

In some embodiments, the size of the oscillation enhancers, such as holes or bubbles, such as gas bubbles, in the foam-like medium may be dependent on the materials used. Hereby, the size of the oscillation enhancers may be dependent on the stirring, in particular whipping, duration and the intensity thereof. Thus, in order to test which composition suits the applied excitation field best, different whipping durations and whipping intensities are tested per composition. The thus generated foam-like media comprising the magnetic objects may then be tested as to it susceptibility to a particular excitation field of a particular excitation frequency. For a particular excitation frequency, the best-performing composition is used.

In this case, the marker device functions on the concept that, upon incidence of the externally applied excitation field, the foam-like medium comprising the magnetic objects situated near at or on the surfaces of the oscillation enhancers begins to oscillate in response to the excitation field. Particularly, the oscillation enhancers begin to oscillate. This causes the magnetic objects to be moved, in particular tilted, which then leads to a change in the magnetic field which can then be read out by a respective reading system.

In some embodiments, the foam-like medium may be provided in solid form. In some embodiments, the foam-like medium may particularly be provided by using a patterned rubber foil made of a solid, rubbery material, such as a polysiloxane/silicone rubber. In these embodiments, although a solid material is used for the foam-like medium, the material is particularly chosen as to be flexible enough to respond to the externally applied excitation field.

In these embodiments, the rubber foil may have at least one hole, particularly a plurality of holes. The magnetic objects, such as magnetic beads or magnetic powder may then be inserted into the holes. Subsequently, the arrangement may be closed up using a respective silicone rubber film or a layer of curing silicone resin having such a wetting that the holes are not filled by the resin. In this case, the oscillation enhancers may thus be provided in terms of holes in the silicone rubber and the magnetic objects may be provided within these holes. In this case, the marker device functions on the concept that, upon incidence of the externally applied excitation field, the foam-like medium including the holes starts oscillating. This causes the magnetic objects arranged in the cavity caused by the holes in the solid foam-like medium to start bouncing around, thus changing their orientation. This leads to the change in magnetic field.

While the above-described embodiments employing a solid material may have a slightly smaller signal and may thus have a slightly reduced efficiency, they bring forward the benefit of being easier to handle since, due to the material being solid, the problem of melting and/liquidity does not occur for these embodiments. As a further benefit, the size of the oscillation enhancers may be defined more easily in these embodiments.

In both cases, i.e. in the case were a liquid/viscous material, such as a foam, is used as the foam-like medium and the case were a solid is used, the benefit resides in the fact that the marker devices of this kind are relatively simply to manufacture as the manufacturing process only requires mixing of a liquid and a plurality of magnetic objects and then stirring, in particular whipping, them for a certain duration and with a certain intensity.

In some embodiments, the at least one magnetic object may be made of a ferromagnetic material.

In this context, it shall be understood that the term ferromagnetic is used to distinguish the magnetic material from paramagnetic materials which do not exhibit a high magnetic force. As such, any magnetic material that is capable of exhibiting a high magnetic force in response to being moved, in particularly tilted or moved around in a cavity, shall be considered a ferromagnetic material in this context. It shall be understood that, for the purpose of this approach, mainly magnetic materials shall be used which have fixed dipole moment, such as to avoid any additional components adding to the change in magnetic field that is determined in order to track the marker device.

As such, the term ferromagnetic shall be understood as referring to a magnetic material that is ferromagnetic somewhere below 200 Kelvin. This is the case since, in accordance with this approach, such a material, being ferromagnetic at below 200 Kelvin, is still usable for the purpose of the approach taught herein, even if the temperature is above its Curie temperature.

In some embodiments, the foam-like medium may comprise a plurality of magnetic objects, and an individual one of the plurality of magnetic objects may have an object size between 4 µm³ to 40 µm³.

In this context, the term object size may particularly refer to the size of one single magnetic object. The specified object size is particularly suitable for having the magnetic objects attach to the surface of the oscillation enhancers in the marker device and to be moved by the respective oscillation. In some embodiments, the plurality of magnetic objects may correspond to a plurality of magnetic beads having the above-indicated size. In some embodiments, the plurality of magnetic objects may correspond to a plurality of magnetic particles, such as particles of a magnetic powder. Further kinds of magnetic objects may be envisioned which allow for the object size being as indicated herein above.

In some embodiments, the body further comprises a material having a melting point below 35°C. In some embodiments, the material comprises a purified mineral oil.

In some embodiments, in particular those where a liquid/viscous material is used as a basis for the marker device, the melting point of the material that the foam-like medium comprises may particularly be below 35°C, i.e. slightly below body temperature of a grown human. This temperature is below average room temperature of between 20°C to 24°C. Accordingly, the marker devices may be stored relatively easily with the foam-like medium being in a solid state. This allows for longer storage times, i.e. maximizes shelf life.

Once employed for usage in tracking the medical device, the surrounding body temperature of around or above 35°C causes the foam-like medium to melt. In the molten state, the oscillation caused by the external excitation field is enhanced. Further, the magnetic objects dispersed in the foam-like medium may move more easily, thereby improving the tracking result.

Thus, providing the foam-like medium with a melting point below 35°C allows for improving the accuracy of the tracking of the marker device.

In some embodiments, the foam-like medium comprises a rubber-like medium.

In some embodiments, the foam-like medium may also be made from a solid material having a certain flexibility. It has been found that a rubber-like medium may particularly be used for this purpose.

In some embodiments, the rubber-like medium may particularly comprise of polysiloxane, also known as silicone. In some embodiments, a polysiloxane rubber may particularly be used.

In these embodiments, the polysiloxane rubber may be provided with respective holes as oscillation enhancers. In these holes, a plurality of magnetic objects may be provided. In order to provide sufficient tracking efficiency. The rubber material should be selected such that the material starts oscillating in response to an externally applied excitation field, in particular an externally applied ultrasound field. If this is the case, the magnetic objects provided in the holes of the rubber will be moved inside these holes. This movement results in a change in magnetic field that may be detected by a respective magnetic response detector. On the basis of this detection, the position of the marker device and, hence, the medical device, may be determined.

In some embodiments, the body may further comprise a coating layer (104). In some embodiments, the coating layer may have a thickness of between 5 to 10 µm. In some embodiments, the coating layer may comprise a parylene coating.

In this context, the term coating layer may particularly refer to a layer arranged on the outer surface of the body comprising the foam-like material.

In some embodiments, the coating layer may particularly act as a sealing layer, forming the outer surface of the body comprising the foam-like medium. This may be particularly useful in cases where the foam-like medium transforms from a viscous/solid to a molten - liquid - state due to the temperature exceeding the foam-like medium's melting point. In these cases, the coating layer may prevent that the foam-like medium exits the body. However, the coating layer may also be used for other purposes, such as protecting the foam-like medium from outer influences during storage and/or attachment to the medical device or the like.

In some embodiments, the layer may particularly have a thickness between 5 to 10 µm and a sufficient resilience to pressure from the outside. In some embodiments, this coating may particularly be made of a polymere having a backbone consist of para-benzenediyl rings connected by 1,2-ethanediyl bridges, commonly known as parylene. The benefit of parylenes as coating is that parylene is bio-compatible and easy to apply in thin layers and brings forward the necessary resilience.

In some embodiments, the body may further comprise a thickening agent.

The term thickening agent may particularly refer to an agent which allows to adjust the flexibility and/or viscosity of the foam-like medium when being mixed with the remaining substances. This may allow to improve efficiency.

In some embodiments, the thickening agent may be used to improve the stability of the foam-like medium, and, thus, in some cases, the body. That is, in particular in those embodiments where are a mixture of liquid and foaming agent is used as basis for the foam-like medium. In these cases, the thickening agent may be added to the liquid and foaming agent in order to improve the stability of the foam-like medium, such that the foam-like medium, in the molten state, is still sufficiently thick such that the relative places of the oscillation enhancers and magnetic objects are maintained. This allows to avoid that the magnetic objects cluster together.

Hereby, the amount of thickening agent should be kept as low as possible in order to avoid damping of the motion of the magnetic objects, while at the same time being as high as needed to keep the relative places of the oscillation enhancers, such as the bubbles, and the magnetic objects. In some embodiments, the thickening agent may particularly correspond to an aluminum salt of fatty acids.

According to another aspect, a reading system for wirelessly tracking a marker device as described above is provided. The reading system comprises an excitation field generator for generating an excitation field for inducing an oscillation in the foam-like medium, a magnetic response detector for detecting the motion of the magnetic objects and generating a respective response signal on the basis of the motion of the magnetic objects and a processor for processing the response signal to track a position of the marker device. In some embodiments, the reading system may also comprise the marker device itself.

In some embodiments, the excitation field generator may be adapted to generate an ultrasound field as the excitation field. In some embodiments, the magnetic response detector may comprise one or more magnetic coil arrangements.

According to another aspect of the invention, a reading system is provided which is used to track the marker device as discussed herein above. The reading system comprises the excitation field generator which may generate the excitation field that causes the oscillation of the foam-like medium. The excitation field generator may hereby particularly be capable of generating an ultrasound field, i.e. may be a generator for generating ultrasound waves. These ultrasound waves generate an ultrasound excitation field which ultrasound energy is transferred to the foam-like medium and causes it to oscillate. The benefit of such an ultrasound generator is such that ultrasound is generally harmless to the human body and, thus, no additional health concerns arise. Further, ultrasound generators are generally available in medical environments due to their large field of application.

Further, the reading system shall comprise a magnetic response detector which is configured to detect the motion of the magnetic objects. This detection of the motion may particularly be performed by detecting the change in the magnetic field generated by the magnetic objects. That is, if the magnetic objects are moved, in particular tilted and/or moved around in the cavities, the magnetic field changes accordingly. Further, since typically hard magnetic materials may be employed, which have a fixed dipole moment, the change in magnetic field is only caused by the tilting of the magnetic objects. Accordingly, it is possible to determine the motion of the magnetic objects using a magnetic response detector and, therefore, draw conclusions on the position of the marker device. Further, in case soft magnetic objects are alternatively or additionally used, the component caused by the change in dipole moment may be corrected for by using an additional magnetic reference field that allows to distinguish the individual components adding to the change in magnetic field as described above.

For the tracking purpose, the magnetic response detector is further configured to, upon detection the motion of the magnetic objects in the above-described manner, generate a respective response signal on the basis of the motion of the magnetic objects. This response signal is then processed by a respective processor to determine the position of the marker device. In some embodiments, this may particularly be achieved by determining the position where a change in magnetic field occurs. At that particular position, the external excitation field acts on the marker device and causes it to oscillate, which, in turn, causes the magnetic objects to be moved, in particular titled, thereby resulting in the magnetic field being changed. For all positions, at which no marker device is provided, no oscillation and, hence, no change in magnetic field may be detected.

According to yet another aspect of the invention, a method for manufacturing a marker device as described herein above is provided. The method comprises the steps of providing a foam-like medium, introducing at least one demagnetized magnetic object into the foam-like medium and magnetizing the at least one demagnetized magnetic object upon introduction.

The providing of the foam-like medium may particularly comprise manufacturing the foam-like medium. For this purpose, a liquid may be mixed with a foaming agent.

In order to introduce the magnetic objects, a plurality of magnetic objects may first be demagnetized. In this context, the term demagnetized shall particularly be considered as referring to a magnetic object that still has magnetic properties, but the magnetic dipoles inside the magnetic object are arranged relative to one another, such that the outside field is low. It shall be understood that the demagnetizing (and subsequent magnetizing) may particularly be performed for hard magnetic materials. However, soft magnetic materials may also be demagnetized and, later on, be magnetized again.

Subsequently, the plurality of demagnetized magnetic objects may be added to the liquid foaming agent mixture. The composition may be stirred, in particular whipped, with a particular intensity and for a particular duration that has previously been determined as beneficial for said case. During stirring, the oscillation enhancers in terms of respective bubbles may be formed. The demagnetized magnetic objects may hereby preferably be provided such as to concentrate on the surfaces of the oscillation enhancers. In some embodiments, a thickening agent may be added to the composition in order to enhance stability.

The thus generated foam-like medium comprising the demagnetized magnetic objects may subsequently be left to change to solid state. Then, a thin coating layer of about 5 to 10 µm may be provided on the foam-like medium.

The demagnetized magnetic objects shall be magnetized upon introduction. This shall be interpreted that meaning that the demagnetized magnetic object may be magnetized right after the marker device is manufactured and prior to being stored. Alternatively, upon introduction may also mean that the magnetizing of the demagnetized magnetic object is done after storage prior to attaching the marker device to the medical device. Further alternatively, the term upon introduction may also refer to the case where the magnetizing of the demagnetized magnetic material is done after the marker device has been attached to the medical device. In some embodiments where soft magnetic materials are used, the magnetizing may even be performed after the medical device having the marker device attached thereto has been inserted into the body. Further possibilities are also foreseeable.

In an even further aspect, a method for tracking a marker device is provided, said method comprising the steps of providing a marker device having a body comprising a foam-like medium and at least one magnetic object arranged in contact with the foam-like medium, generating, by an excitation field generator, an excitation field for inducing an oscillation in the foam-like medium, detecting, by a magnetic response detector, a motion of the magnetic objects, generating a respective response signal on the basis of the motion of the magnetic objects, and processing the response signal to track a position of the marker device. It shall be understood that the steps of the tracking method may be performed by a respective computer program, which, when implemented by programming code means on a processing device, causes the processing device to implement the method steps.

According to yet another aspect, a computer program is provided, the computer program for controlling a reading system for tracking a marker device as specified herein above, when executed by a processing device, is adapted to perform the method as specified herein above. In an even further aspect, a computer-readable medium having stored thereon the computer program is provided.

It shall be understood that the marker device of claim 1, the reading system of claim 11, the manufacturing method of claim 12 and the tracking method of claim 13, the computer program according to claim 14 and the computer-readable medium of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a marker device, and
Fig. 2 shows schematically and exemplarily an embodiment of a reading system for tracking the marker device.

### DETAILED DESCRIPTION OF EMBODIMENTS

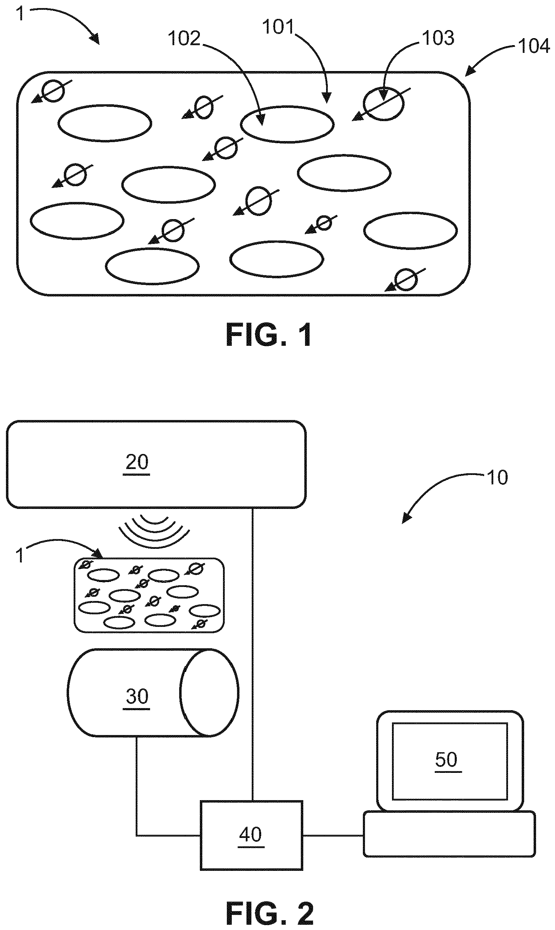

Fig. 1 schematically and exemplarily shows an embodiment of a marker device 1 for being attached to a medical device for being tracked by a reading system, for example during local anesthesia on a human being, in particular a patient. The marker device 1 comprises a foam-like medium 101, a plurality of oscillation enhancers 102, a plurality of magnetic objects 103 and a coating layer 104.

In the specific embodiment according to Fig. 1, the marker device 1 is provided as a miniature patch attachable to the medical device. The foam-like medium 101 and the coating layer 104 form the body of the miniature patch. That is, in the specific embodiment according to Fig. 1, the body consists of the foam-like medium 101 and the coating layer 104.

The foam-like medium 101 comprises a plurality of oscillation enhancers 102, which, in the specific embodiment according to Fig. 1 correspond to bubbles in the foam-like medium 101. The oscillation enhancers 102 have a predetermined size. That is, the size value of the individual oscillation enhancers 102 is within a certain predetermined range. The range in which the size value may be may hereby particularly be predetermined on the basis of the excitation frequency of the externally applied excitation field that is supposed to act upon the marker device 1 for the purpose of tracking. Specifically, the size should be chosen such that the oscillation enhancers 102 have a resonance frequency of oscillation that is close to the excitation frequency of the externally applied excitation field.

Further, the foam-like medium 101 comprises a plurality of magnetic objects 103, which, in the specific embodiment of Fig. 1, are provided in terms of a plurality of magnetic beads having a size between 5 and 30 µm.

In the specific embodiment of Fig. 1, the plurality of magnetic objects 103 comprises a plurality of hard magnetic objects, i.e. magnetic objects having a fixed dipole moment as indicated in Fig. 1 by the arrows. The plurality of magnetic objects 103 is dispersed in the foam-like medium 101. Although not explicitly shown in the embodiment according to Fig. 1, the plurality of magnetic objects preferably concentrate around the individual oscillation enhancers 102 of the plurality of oscillation enhancers 102, in particular on the surfaces thereof. This allows to maximize the motion of the magnetic objects 103 upon oscillation of the foam-like material and, thereby, improve the overall signal quality of the response of the marker device 1.

In the embodiment according to Fig. 1, the foam-like medium 101 has been prepared by mixing a purified mineral oil product having a melting point slightly below body temperature, i.e. slightly below 35°C, with a thickening agent made of an aluminum salt of fatty acids. To this mixture, magnetic beads of a size between 5 to 30 µm may then be added. These magnetic beads may be made of any hard magnetic and/or ferromagnetic material. In some embodiments, the magnetic beads may be added by adding a crushed magnet powder, such as a crushed neodymium powder.

This composition may then be stirred with a particular intensity and for a particular duration, until bubbles are being formed in the composition. Then, the composition may be left to change into a solid state. Since the melting point of the purified mineral oil used is slightly below body temperature, i.e. below 35°C, the composition will become solid upon cooling to room temperature. In the solid state, the coating 104, acting as a protective layer, may be added. The coating 104 according to Fig. 1 has a thickness of around 5 µm. For this purpose, in the specific embodiment according to Fig. 1, this coating comprises or consists of a parylene. The advantage of using parylene is that it is bio-compatible and easy to apply in thin layers.

It is noted that, although in the specific embodiment according to Fig. 1, the foam-like medium 101 has been prepared using a purified mineral oil having a melting temperature slightly below 35° mixed with an thickening agent, and, thereby, becomes liquid once used attached to the medical device, it shall be understood that the foam-like medium 101 of the marker device 1 may also be manufactured such as to comprise a solid foam-like medium. In particular, in some embodiments, the foam-like medium may particularly be manufactured from a patterned rubber foil made of a polysiloxane - silicone - rubber, as this kind of rubber is flexible enough to respond to the externally applied excitation field.

In order to manufacture the marker device 1, the silicone rubber foil may be provided with a plurality of holes, i.e. cavities in the foil. Hereby, the magnetic objects, such as magnetic beads or magnetic powder, are inserted into the cavities. Upon insertion, the cavities may be closed off, using a cover, such as a silicone rubber film or a layer of curing silicone resin. In such a case, the oscillation enhancers correspond to the holes or cavities in the closed rubber patch, whereby the magnetic objects are provided in the holes.

During manufacture of the marker device 1, the plurality of magnetic objects 103 may be added in a magnetized or in a demagnetized state. If the plurality of magnetic objects 103 is added in a demagnetized state, this means that the magnetic dipoles in each of the magnetic objects 103 are arranged relative to one another such that the overall magnetic field of the particular magnetic object 103 is low. In this case, the plurality of magnetic objects 103 has to be magnetized again later on. This may be done right after manufacture and before storing the thus manufactured marker devices 1 or directly prior to or upon attachment of the marker device 1 to the medical device. In some cases, the magnetizing may even be done upon introduction of the medical device into the human being, in particular the patient.

The benefit of having the marker device 1 being made of a foam-like medium 101 that has a melting point slightly below 35°C, i.e. slightly below body temperature of a grown human being, is such that, the marker device 1 may be stored at room temperature with the foam-like medium 101 being in a solid state, which maximizes shelf life. On the other hand, once the marker device 1 is introduced into the human body for the sake of providing tracking of the medical device, the foam-like medium melts. Since thickening agent has been added to the foam-like medium 101, the foam-like medium 101 then corresponds to a thickened liquid, having a plurality of oscillation enhancers 102 and a plurality of magnetic objects 103 also provided therein. Due to the increased viscosity caused by the thickening agent, the plurality of oscillation enhancers 102 and the plurality of magnetic objects 103 may remain at a substantially same relative position in the marker device 1 during the tracking. This further improves accuracy.

Thus, using the marker device 1 according to Fig. 1, a tracking of a medical device may be achieved with high accuracy.

Fig. 2 schematically and exemplarily shows an embodiment of a reading system 10 for tracking a marker device 1 as discussed herein above. The reading system 10 comprises an excitation field generator 20, a magnetic response detector 30, a processor 40 and a user interface 50, comprising a display unit and a user input unit.

In the specific embodiment according to Fig. 2, the marker device 1 as described in relation to Fig. 1 is used to track a medical device (not shown) during an invasive procedure, which, in the specific embodiment according to Fig. 2, corresponds to the application of a local anesthesia using a needle as medical device. For this purpose, the marker device 1 is attached to the needle as the medical device to be tracked. Then, the excitation field generator 20 is used to generate an externally applied excitation field. In the specific embodiment according to Fig. 1, this externally applied excitation field corresponds to an ultrasound excitation field generated by an ultrasound generator.

The externally applied excitation field generated by the excitation field generator 20 causes the foam-like medium 101 in the marker device 1 to oscillate in response thereto. Since, as described above, the foam-like medium 101 is in a viscous state when attached to the medical device and used during the invasive procedure, this oscillation is improved. The oscillation of the foam-like medium 101 causes the plurality of oscillation enhancers 102 to oscillate. Since the size of each of the plurality of oscillation enhancers 102 has been determined such that the oscillation occurs with the resonance frequency of the excitation field, this further enhances the oscillation effect. The plurality of magnetic objects 103 which are dispersed in the foam-like medium 101 and, in the specific embodiment according to Fig. 2, concentrate at the surfaces of the oscillation enhancers 102 are caused to move due to this oscillation. In the specific embodiment according to Fig. 2, the motion of the plurality of magnetic objects 103 particularly corresponds to a tilting motion.

The tilting of the plurality of magnetic objects 103 causes the fixed dipole moments to change direction relative to the magnetic response detector 30. The magnetic response detector 30 thus detects a change in the magnetic field that is indicative of a motion, i.e. a tilting in this case, of the magnetic objects 103. Based on this detected motion, the magnetic response detector 30 may then generate a respective response signal indicating that the plurality of magnetic objects 103 has been moved.

It is noted that, although in the specific embodiment according to Fig. 2, the magnetic objects 103 are caused to tilt in response to the externally applied excitation field, in further embodiments, in particular those in which the foam-like medium 101 comprises a solid and the magnetic objects 103 are provided in the holes/cavities, the magnetic objects 103 may be caused to move within the holes/cavities, to bounce of the walls of the holes/cavities. This movement likewise causes a change in the magnetic field detected by the magnetic response detector 30.

The magnetic response detector 30 may then provide the response signal to the processor 40. Processor 40 uses the response signal to track the medical device to which the marker device 1 is attached.

Hereby, the signal processing by processor 40 is performed in a similar manner as known approaches employing PVDF. Hereby, the person to be examined, i.e. the patient, into whom the medical device is introduced is scanned by the ultrasound system using the excitation field generator 20. The magnetic response detector 30 detects the change in magnetic field. The moment, the excitation field generator 20 is at a scanning position where the marker device 1 is positioned, the magnetic response detector may detect an increased signal. The processor 40 receives the information about the scanning position from the ultrasound system comprising the excitation field generator 20 and the signal from the magnetic response detector, this allows the processor to precisely determine the position of the marker device 1 (based on the highest signal being measured there) in the obtained ultrasound image. Hereby, the depth is generated from a relative timing between the ultrasound system comprising the excitation field generator 20 and the magnetic response detector 30. That is, the magnetic response detector 30 gives a signal at exactly half the time the corresponding acoustic echo in in the ultrasound system comprising the excitation field generator 20 is obtained.

That is, the processor 40 makes use of the fact that the change in magnetic field detected by the magnetic response detector 30 will depend upon the position of the marker device 1 relative to the excitation field generator 20 and the magnetic response detector 30. That is, if the marker device 1 is positioned such that it does not experience the excitation field generated by the excitation field, the magnetic response detector 30 cannot detect any change. Only if the foam-like medium 101 in the marker device 1 starts oscillating in response to the externally applied excitation field, a response may be detected by the magnetic response detector 30. The processor 40 may thus process the response signal provided by the magnetic response detector 30 and determine the position of the marker device 1 and, thereby, the position of the medical device. The processor 40 may then be configured to provide the processing result to user interface 50. The display of user interface 50 may use the information to generate a graphical representation of the tracking and present said graphical representation to a user, such as a doctor. This allows the user, in particular the doctor, to track the position of the medical device during the invasive procedure in real time and, to intervene if necessary.

That is, in accordance with the invention, a marker device is provided which allows to track the position of a medical device with high accuracy using a magnetic field that is generated through movement of a plurality of magnetic objects in a viscous foam-like medium that is caused to oscillate by respective ultrasound waves. The movement causes a change in the magnetic field generated by the plurality of magnetic objects. This change may be picked up by a magnetic response detector. The thus obtained response signal may be used to determine the position of the marker device 1 and, hence, the medical device, during an invasive procedure.

As such, the current approach allows to track the medical device with high accuracy, while at the same time keeping the costs for the tracking low and the tracking in itself simple and harmless for the patient.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the generating of the excitation field, the detecting of the motion of the magnetic objects, the generating of a response signal and/or the processing of said response signal, et cetera, performed by one or several units or devices can be performed by any other number of units or devices. These procedures, particularly the tracking of the marker device in accordance with the tracking method as carried out by the reading system, can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a marker device to be tracked, wherein the marker device comprises a body comprising a foam-like medium and at least one magnetic object arranged in contact with the foam-like medium, wherein the foam-like medium is adapted to oscillate in response to an externally applied excitation field, such as to initiate a motion of the at least one magnetic object.

## Claims

1. A marker device (100) to be tracked, wherein the marker device (100) comprises:
a body comprising a foam-like medium (101) and at least one magnetic object (103) arranged in contact with the foam-like medium (101);
wherein the foam-like medium (101) is adapted to oscillate in response to an externally applied excitation field, such as to initiate a motion of the at least one magnetic object (103).

2. The marker device (100) according to claim 1, wherein the foam-like medium (101) comprises one or more oscillation elements (102) having a predetermined size.

3. The marker device (100) according to anyone of claims 1 and 2, wherein the at least one magnetic object (103) is made of a ferromagnetic material.

4. The marker device (100) according to anyone of claims 1 to 3, wherein the foam-like medium comprises a plurality of magnetic objects (103), and
wherein an individual one of the plurality of magnetic objects has an object size between 4 µm³ to 40 µm³.

5. The marker device (100) according to anyone of claims 1 to 4, wherein the body further comprises a material having a melting point below 35°C.

6. The marker device (100) according to anyone of claims 1 to 5, wherein the foam-like medium comprises a purified mineral oil.

7. The marker device according to anyone of claims 1 to 6, wherein the foam-like medium comprises a rubber-like medium.

8. The marker device (100) according to anyone of claims 1 to 7, wherein the body further comprises a coating layer (104).

9. The marker device according to claim 7, wherein the coating layer (104) has a thickness of between 5 to 10 µm.

10. The marker device according to anyone of claims 1 to 7, wherein the body further comprises a thickening agent.

11. A reading system (10) for wirelessly tracking a marker device (100) according to anyone of claims 1 to 10, the system comprising:
an excitation field generator (20) for generating an excitation field for inducing an oscillation in the foam-like medium (10),
a magnetic response detector (30) for detecting the motion of the magnetic objects (103) and generating a respective response signal on the basis of the motion of the magnetic objects (103);
a processor (40) for processing the response signal to track a position of the marker device.

12. A method for manufacturing a marker device (100) according to anyone of claims 1 to 10, the method comprising:
providing a foam-like medium (101),
introducing at least one demagnetized magnetic object (103) into the foam-like medium, and
magnetizing the at least one demagnetized magnetic object upon introduction.

13. A method for tracking a marker device (100), comprising:
providing a marker device having a body comprising a foam-like medium (101) and at least one magnetic object (103) arranged in contact with the foam-like medium (101);
generating, by an excitation field generator, an excitation field for inducing an oscillation in the foam-like medium (10),
detecting, by a magnetic response detector (30), a motion of the magnetic objects (103);
generating a respective response signal on the basis of the motion of the magnetic objects (103); and
processing the response signal to track a position of the marker device.

14. A computer program for controlling a reading system according to claim 11 to track a marker device according to anyone of claims 1 to 10, which, when executed by a processing device, is adapted to perform the method according to anyone of claim 13.

15. A computer-readable medium having stored thereon the computer program according to claim 14.
